(19) Europäisches Patentamt
European Patent Office
Office européen des brevets

(11) Veröffentlichungsnummer: **0 133 274**
**A1**

(12) # EUROPÄISCHE PATENTANMELDUNG

(21) Anmeldenummer: **84108722.4**

(22) Anmeldetag: **24.07.84**

(51) Int. Cl.[4]: **C 07 C 125/06**

(30) Priorität: **02.08.83 DE 3327824**

(43) Veröffentlichungstag der Anmeldung: **20.02.85 Patentblatt 85/8**

(84) Benannte Vertragsstaaten: **AT BE CH DE FR GB IT LI NL SE**

(71) Anmelder: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Towae, Friedrich, Dr., Schwedlerstrasse 118, D-6700 Ludwigshafen (DE)**

(54) **Verfahren zur Herstellung von N,O-substituierten Mono- und/oder Polyurethanen.**

(57) Die Erfindung beschreibt ein Verfahren zur Herstellung von N,O-substituierten Mono- und/oder Polyurethanen der Formel

$R^1[NHCOOR^2]_n$,

in der
$R^1$ einen gegebenenfalls substituierten aliphatischen, cycloaliphatischen, aromatischen, araliphatischen oder heterocyclischen Rest,
$R^2$ einen gegebenenfalls mit Alkoxy- oder Polyoxyalkylengruppen substituierten aliphatischen, cycloaliphatischen oder araliphatischen Rest und
n eine ganze Zahl von 1 bis 5 bedeuten,
durch Umsetzung von N-substituierten Allophansäure- und/oder Polyallophansäureestern mit Alkoholen in Abwesenheit oder Gegenwart von Katalysatoren bei Temperaturen von mindestens 160°C, vorzugsweise 165 bis 250°C.

EP 0 133 274 A1

ACTORUM AG

Verfahren zur Herstellung von N,O-substituierten Mono- und/oder Polyurethanen

Urethane werden technisch üblicherweise durch Umsetzung von Isocyanaten mit Alkoholen oder von Chlorkohlensäureestern mit Aminen hergestellt, wobei sowohl die Isocyanate als auch die Chlorkohlensäureester durch Phosgenierung der entsprechenden Amine und Abspaltung von Chlorwasserstoff oder durch Phosgenierung der Alkohole gewonnen werden (Methoden der organischen Chemie, Houben-Weyl, Band 8, Seiten 137, 120 und 101, Georg Thieme Verlag, Stuttgart, 1952). Diese Verfahren sind technisch sehr aufwendig; ferner bringt die Verwendung von Phosgen wegen der damit verbundenen Sicherheits- und Umweltschutzmaßnahmen erhebliche Nachteile mit sich.

In jüngerer Zeit wurden daher eine Reihe von Verfahren zur Herstellung von Urethanen beschrieben, bei denen Kohlensäurederivate mit primären Aminen in Gegenwart von Alkoholen zur Reaktion gebracht werden.

Nach Angaben der EP-A-18 581 (CA-PS 1.121.373) werden Aryl-mono- und/oder -polyurethane durch Umsetzung von O-Alkylcarbamidsäureestern mit primären aromatischen Mono- und/oder Polyaminen in Gegenwart von Alkoholen und gegebenenfalls Harnstoff hergestellt. Als Kohlensäurederivate werden in der EP 66 231 N,N'-Diarylharnstoffe und in der EP 48 371 Dialkylcarbonate genannt.

In Methoden der organischen Chemie, Houben-Weyl, Band 8, wird ferner die Alkoholyse von unsubstituierten Allophansäureestern der Formel $H_2N$-CO-NH-CO-OR zu N-unsubstituierten Carbaminsäureestern erwähnt (Seite 206). Für die Herstellung von N-substituierten Urethanen wird keine technische Lehre vermittelt.

Die EP 59 400 beschreibt ein Verfahren zur Herstellung von N,O-disubstituierten Urethanen durch Umsetzung von primären Aminen und Alkoholen mit Allophansäureestern oder Allophansäureester aufweisenden Gemischen aus Allophansäureestern, Harnstoff und/oder Carbamidsäureestern bei Temperaturen von 130 bis 350°C. Für die Durchführbarkeit der Umsetzung ist die Gegenwart von Aminen unbedingt erforderlich, was auch durch die Beispiele zum Ausdruck kommt.

Überraschenderweise wurde nun gefunden, daß man N,O-substituierte Mono- und/oder Polyurethane auf einfache Weise und mit sehr guten Ausbeuten her-

M/P

stellen kann, wenn man N-substituierte Allophansäure- und/oder Polyallophansäureester alkoholysiert.

Gegenstand der Erfindung ist somit ein Verfahren zur Herstellung von N,O-substituierten Mono- und/oder Polyurethanen der Formel

$$R^1[NHCOOR^2]_n ,$$

in der

$R^1$ einen gegebenenfalls substituierten aliphatischen, cycloaliphatischen, aromatischen, araliphatischen oder heterocyclischen Rest,

$R^2$ einen gegebenenfalls mit Alkoxy- oder Polyoxyalkylengruppen substituierten aliphatischen, cycloaliphatischen oder araliphatischen Rest, die gleich oder verschieden sein können, falls n größer als 1 ist, und

n eine ganze Zahl von 1 bis 5 bedeuten,

das dadurch gekennzeichnet ist, daß man N-substituierte Allophansäure- und/oder Polyallophansäureester mit Alkoholen $R^2OH$ in Abwesenheit oder vorzugsweise Gegenwart von Katalysatoren bei Temperaturen von mindestens 160°C zur Reaktion bringt.

Das erfindungsgemäße Verfahren ermöglicht in vorteilhafter Weise die bei der thermischen Spaltung von Urethanen zu Isocyanaten als Nebenprodukte anfallenden N-substituierten Allophansäure- und/oder Polyallophansäureester in Urethane überzuführen und so erneut der Isocyanatherstellung zugänglich zu machen.

Als Ausgangskomponente geeignete Allophansäure- und/oder Polyallophansäureester, die für einwertige Reste $R^1$ durch die Formel

$$R^1-NH[CONR^1]_x-COOR^3,$$

in der $R^1$ die obengenannte Bedeutung besitzt, $R^3$ einen gegebenenfalls substituierten aliphatischen, cycloaliphatischen, aromatischen, araliphatischen oder heterocyclischen Rest bedeutet, der vorzugsweise dem Rest $R^2$ entspricht und x eine ganze Zahl von mindestens 1, vorzugsweise 1 bis 4 ist, beispielhaft charakterisiert werden können, können nach bekannten Verfahren erhalten werden. Genannt sei beispielsweise die Acylierung von Alkoholen mit substituiertem Harnstoffchlorid oder substituiertem Allophansäurechlorid oder die direkte Einführung der Carbonestergruppe in substituierte Harnstoffe mit Kohlensäuredialkylester oder Chlorkohlensäurealkylester. Die substituierten Allophansäure- und/oder Polyallophansäureester werden vorzugsweise jedoch durch Umsetzung von Urethanen oder Alkoholen mit einem oder mehreren Mol(en) Isocyanat hergestellt oder fallen, dieser Reaktion entsprechend, insbesondere bei der thermischen Spaltung von Urethanen zu Isocyanaten als Nebenprodukte an.

Als Alkohole $R^2OH$ für das erfindungsgemäße Verfahren eignen sich lineare oder verzweigte Alkanole mit 1 bis 18, vorzugsweise 1 bis 10 und insbesondere 3 bis 6 Kohlenstoffatomen, mit Alkoxygruppen mit 1 bis 4 Kohlenstoffatomen oder Polyoxyalkylengruppen substituierte lineare oder verzweigte Alkanole mit 2 bis 24 Kohlenstoffatomen, vorzugsweise 4 bis 8 Kohlenstoffatomen, cycloaliphatische Alkohole mit 4 bis 12 Kohlenstoffatomen, vorzugsweise 6 bis 8 Kohlenstoffatomen und/oder araliphatische Alkohole mit 7 bis 15 Kohlenstoffatomen, vorzugsweise 7 bis 10 Kohlenstoffatomen. Im einzelnen seien beispielhaft genannt: Methanol, Ethanol, Propanol, iso-Propanol, Butanol, iso-Butanol, Pentanol, iso-Pentanol, Hexanol, iso-Hexanol, Heptanol, iso-Heptanol, Octanol, iso-Octanol, Nonanol, iso-Nonanol, Decanol, iso-Decanol, Dodecanol, 2-Ethylhexanol, 2-Ethylbutanol, Hexadecanol, Octadecanol, 2-Methoxyethanol, 2-Ethoxyethanol, 2-Propoxyethanol, 2-Butoxyethanol, 2-(2-Methoxy-ethoxy)-ethanol, 2-(2-Butoxyethoxy)-ethanol, Cyclopentanol, Cyclohexanol, Methylcyclohexanole, Cyclohexylmethanol, 3,3,5-Trimethylcyclohexanol, 4-tert.-Butylcyclohexanol, 2-Hydroxydecalin, Borneol, Isoborneol, 2-Hydroxyethoxybenzol, Benzylalkohol, 2-Phenylethanol, 2-(Methoxyphenoxy)-ethanole, 1-Phenylethanol, 3-Phenyl-1-propanol, 4-Methoxybenzylalkohol.

Besonders bewährt haben sich und daher vorzugsweise verwendet werden als Alkohole Methanol, Ethanol, n-Propanol, iso-Propanol, n-Butanol, iso-Butanol, n-Pentanol, Hexanol, Heptanol, Octanol, Nonanol, Decanol, Ethylhexanol, Cyclopentanol, Benzylalkohol, Cyclohexanol und 2-Phenylethanol. Die Alkohole können einzeln oder in Form von Mischungen verwendet werden.

Da die Alkohole nach dem erfindungsgemäßen Verfahren vorzugsweise in einem molaren Überschuß eingesetzt werden, kann üblicherweise auf den Zusatz von Lösungsmitteln verzichtet werden. Bei Verwendung spezieller Alkohole kann es dennoch zweckmäßig sein, diese mit unter den Reaktionsbedingungen inerten, unpolaren oder vorzugsweise polaren Lösungsmitteln oder -mischungen mit Siedepunkten von 50 bis 350°C zu mischen. Im einzelnen seien als Lösungsmittel beispielsweise genannt: n-Nonan, n-Decan, n-Dodecan, n-Butylcyclohexan, n-Hexylcyclohexan, Decahydronaphthalin, Isopropylbenzol, 1,3-Diethylbenzol, n-Butylbenzol, Chlorbenzol, 4-Chlortoluol, 1,2-Dichlorbenzol, 2,4-Dichlortoluol, 1,2,4-Trichlorbenzol, 2-Chlor-4-isopropyl-1-methylbenzol, Anisol, Cyclohexylethylether, Diethylenglykoldimethylether, Benzylmethylether, 4-Methoxytoluol,

Parachloranisol, Di-n-hexylether, N,N-Dimethylformamid, N,N-Diethylformamid, N-Methylformamid, Dimethylacetamid, N-Methylpyrrolidon, Caprolactam, Tetralin, Sulfolan, Hexamethylphosphorsäuretriamid, Dimethylsulfoxid, Ethylenglykolmonomethyletheracetat, Di-n-propylcarbonat, Cyclohexylacetat, Diisobutylcarbonat, 2-Ethylpyridin, N,N-Dimethyl-2-methylanilin, N,N-Dimethylanilin, N-Methyl-N-ethylanilin, N,N-Dimethyl-2-chloranilin, N,N-Diethylanilin, Chinolin, Nitrobenzol, 2-Nitrotoluol, 2,4-Dimethyl-1-nitrobenzol, Acetonitril, N-Capronitril, Benzonitril, Tolunitril und Phenylacetonitril.

Die Alkoholyse der Allophansäure- und/oder Polyallophansäureester kann in Abwesenheit von Katalysatoren durchgeführt werden. Zur Erhöhung der Reaktionsgeschwindigkeit und damit der Raum-Zeit-Ausbeute hat es sich jedoch als vorteilhaft erwiesen, die Umsetzung in Gegenwart eines oder mehrerer Katalysatoren durchzuführen. Als Katalysatoren eignen sich anorganische und organische Verbindungen, die ein oder mehrere Kationen, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14th. Edition, publiziert von Chemical Rubber Publishing Co. 2310 Superior Ave, N.E. Cleveland, Ohio, enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate. Beispielhaft genannt seien die Kationen folgender Metalle: Lithium, Natrium, Kalium, Magnesium, Calcium, Aluminium, Gallium, Zinn, Blei, Wismut, Antimon, Kupfer, Silber, Gold, Zink, Quecksilber, Cer, Titan, Vanadium, Chrom, Molybdän, Mangan, Eisen, Kobalt und Nickel. Vorzugsweise Verwendung finden die Kationen von Lithium, Calcium, Aluminium, Zinn, Wismut, Antimon, Kupfer, Zink, Titan, Vanadium, Chrom, Molybdän, Mangan, Eisen und Kobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumethanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminium-isobutylat, Aluminiumtrichlorid, Wismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titan-tetrabutanolat, Titantetrachlorid, Titantetraphenolat, Titannaphthenat, Vanadium-(III)-chlorid, Vanadiumacetonylacetat, Chrom--(III)-chlorid, Molybdän-(IV)-oxid, Molybdänacetylacetonat, Wolfram-(VI)--oxid, Mangan-(II)-chlorid, Mangan-(II)-acetat, Mangan-(III)-acetat, Eisen-(II)-acetat, Eisen-(III)-acetat, Eisenphosphat, Eisenoxalat, Eisen--(III)-chlorid, Eisen-(III)-bromid, Kobaltacetat, Kobaltchlorid, Kobaltsulfat, Kobaltnaphthenat, Nickelchlorid, Nickelacetat und Nickelnaphthenat sowie deren Gemische.

Die Katalysatoren werden zweckmäßigerweise in Mengen, die 0,0001 bis 10 Gew.%, vorzugsweise 0,0001 bis 3 Gew.% und insbesondere 0,0005 bis 1 Gew.%, bezogen auf das Gewicht der Allophansäure- und/oder Polyallophansäureester verwendet. Nach einer speziellen Ausführungsform können die Metallkationen auch in heterogener Phase, z.B. gebunden an Ionenaustauscher, in der Reaktionsmischung vorliegen.

Zur Herstellung der N,O-substituierten Mono- und/oder Polyurethane nach dem erfindungsgemäßen Verfahren werden die Allophansäure- und/oder Polyallophansäureester mit Alkoholen, vorzugsweise den den Esteralkoholen entsprechenden Alkoholen, in Mengen pro $\{CO-NR^1\}$-Gruppe von 1 bis 50 Molen, vorzugsweise 3 bis 15 Molen und insbesondere 3 bis 10 Molen zur Reaktion gebracht.

Sofern die den Allophansäure- und/oder Polyallophansäureestern zugrunde liegenden Esteralkohole $R^3OH$ und die verwendeten Alkohole $R^2OH$ nicht identisch sind, findet neben der Alkoholyse auch in mehr oder weniger großem Ausmaße eine Umesterung statt. Diese führt zu Polyurethanen und/oder Polyurethanmischungen mit unterschiedlichen Estergruppen. Durch geeignete Wahl der Ausgangskomponenten, insbesondere der Alkohole $R^2OH$ und -mengen kann jedoch gleichzeitig mit der Alkoholyse eine praktisch vollständige Umesterung erzielt werden. Diese Verfahrensvariante - Alkoholyse und gleichzeitige Umesterung - ist insbesondere dann von Bedeutung, wenn die Mono- und/oder Polyurethane thermisch in Isocyanate und Alkohole gespalten werden und die erhaltenen Spaltprodukte ohne vorhergehende Umesterung nahezu gleiche Siedepunkte aufweisen würden.

Durch eine Verminderung des Alkoholüberschusses kann der Grad der Umesterung reduziert werden, wobei es sich jedoch als zweckmäßig erwiesen hat, insbesondere bei molaren Verhältnissen von $\{CO-NR^1\}$-Gruppen zu Alkoholen von 1:1 bis ungefähr 1:2 in Gegenwart von Lösungsmitteln zu alkoholysieren.

Die Alkoholyse wird bei Temperaturen von mindestens 160°C, vorzugsweise 165° bis 250°C und insbesondere 180° bis 230°C und Drücken von 0,1 bis 120 bar, vorzugsweise 0,5 bis 60 bar, insbesondere 1 bis 40 bar, durchgeführt. Bei gegebener Temperatur wird die Umsetzung dann vorzugsweise unter dem Eigendruck der Reaktionsmischung durchgeführt.

Für den genannten Temperaturbereich ergeben sich Reaktionszeiten von 0,5 bis 100 Stunden, vorzugsweise von 1 bis 50 Stunden, und insbesondere 2 bis 25 Stunden.

Die Mono- und/oder Polyurethane werden nach dem erfindungsgemäßen Verfahren zweckmäßigerweise wie folgt hergestellt. Die Allophansäure- und/oder Polyallophansäureester und Alkohole $R^2OH$ werden in den genannten Mengenverhältnissen gemischt und gegebenenfalls in Gegenwart eines Katalysators und Lösungsmittels in einem Reaktionsgefäß gegebenenfalls unter Rühren erhitzt. Aus der erhaltenen Reaktionsmischung werden anschließend, gegebenenfalls nach Abtrennung des Katalysators und Abfiltrieren von Feststoffen, die Mono- und/oder Polyurethane isoliert, beispielsweise durch Abdestillieren des überschüssigen Alkohols, durch partielles Abdestillieren des überschüssigen Alkohols und Auskristallisieren, durch Ausfällen mit oder auch durch Umkristallisieren aus anderen Lösungsmitteln. Der abgetrennte Alkohol kann gegebenenfalls zurückgeführt werden.

Nach dem erfindungsgemäßen Verfahren können, wie bereits dargelegt wurde, Mono- und/oder Polyurethane hergestellt werden mit der Formel

$$R^1 [NHCOOR^2]_n ,$$

in der bedeuten:

n eine die Wertigkeit kennzeichnende ganze Zahl von 1 bis 5, vorzugsweise 2 bis 3,

$R^1$ in Abhängigkeit von der Wertigkeit n einen 1- bis 5-wertigen, vorzugsweise 2- bis 3-wertigen,
substituierten oder vorzugsweise unsubstituierten aliphatischen Kohlenwasserstoffrest mit 1 bis 18, vorzugsweise 3 bis 10 Kohlenstoffatomen,
substituierten oder vorzugsweise unsubstituierten cycloaliphatischen Kohlenwasserstoffrest mit 3 bis 18, vorzugsweise 6 bis 13 Kohlenstoffatomen,
substituierten oder vorzugsweise unsubstituierten aromatischen Kohlenwasserstoffrest mit 6 bis 15, vorzugsweise 6 bis 10 Kohlenstoffatomen,

einen substituierten oder vorzugsweise unsubstituierten araliphatischen Kohlenwasserstoffrest mit 7 bis 34, vorzugsweise 8 bis 20 Kohlenstoffatomen oder
einen substituierten oder vorzugsweise unsubstituierten 5- bis 6-gliedrigen heterocyclischen Rest, der zusätzlich noch mit einem Benzolring annelliert sein kann, und

$R^2$ einen linearen oder verzweigten Alkylrest mit 1 bis 18, vorzugsweise 1 bis 10 und insbesondere 3 bis 6 Kohlenstoffatomen,
einen mit Alkoxygruppen mit 1 bis 4 Kohlenstoffatomen oder Polyoxyalkylengruppen, beispielsweise Polyoxyethylen- und/oder Polyoxypropylengruppen, substituierten linearen oder verzweigten Alkylrest mit 2 bis 24, vorzugsweise 4 bis 8 Kohlenstoffatomen,
einen substituierten oder unsubstituierten Cycloalkylrest mit 4 bis 12, vorzugsweise 6 bis 8 Kohlenstoffatomen und/oder einen substituierten oder unsubstituierten araliphatischen Kohlenwasserstoffrest mit 7 bis 15, vorzugsweise 7 bis 10 Kohlenstoffatomen.

Als Substituenten für die 1- bis 5-wertigen, aliphatischen und cycloaliphatischen Reste $R^1$ kommen beispielsweise in Betracht: Alkoxygruppen mit 1 bis 6, vorzugsweise 1 bis 4 Kohlenstoffatomen,
Aroxygruppen mit 6 bis 10, vorzugsweise 6 bis 8 Kohlenstoffatomen,
Acylreste mit 2 bis 6 Kohlenstoffatomen,
Alkylmercaptogruppen mit 1 bis 6 Kohlenstoffatomen,
Arylmercaptogruppen mit 6 bis 10 Kohlenstoffatomen,
Alkylcarbonylgruppen mit 1 bis 12 Kohlenstoffatomen,
Dialkylaminogruppen mit 1 bis 10 Kohlenstoffatomen,
Acylaminogruppen mit 1 bis 8 Kohlenstoffatomen,
Arylcarbonylgruppen mit 7 bis 9 Kohlenstoffatomen,
Nitrogruppen, Cyanogruppen und Halogenatome.

Für die 1- bis 5-wertigen araliphatischen und aromatischen Reste $R^1$ seien neben den obengenannten Substituenten für die aliphatischen und cycloaliphatischen Reste $R^1$ zusätzlich noch beispielsweise genannt:

lineare oder verzweigte Alkylreste mit 1 bis 12, vorzugsweise 1 bis 4 Kohlenstoffatomen,
Alkylsulfonsäureester mit 1 bis 10, vorzugsweise 1 bis 4 Kohlenstoffatomen im linearen oder verzweigten Alkylrest und
Sulfonamidgruppen.

Bevorzugte Reste $R^1$ sind solche, die sich beispielsweise von folgenden primären Aminen ableiten:

Aliphatischen und cycloaliphatischen Monoaminen wie z.B. Methylamin, Ethylamin, Propylamin, Isopropylamin, n-Butylamin, sek.-Butylamin, tert.-Butylamin, Isobutylamin, 2- und 3-Methylbutylamin, Neopentylamin, n-Pentylamin, 2-Methyl-pentylamin, sek.-iso-Amylamin, n-Hexylamin, 2-Methylhexylamin, 2-Ethylhexylamin, n-Heptylamin, n-Octylamin, n-Nonylamin, n-Decylamin, n-Dodecylamin, 2-Phenylpropylamin, Benzylamin, Cyclopentylamin, Cyclohexylamin, tert.-Butylcyclohexylamin, aliphatischen Diaminen wie Ethylendiamin, 1,3- und 1,2-Propylendiamin, 2,2-Dimethyl-1,3-propylendiamin, 1,4-Butylendiamin, 1,5-Pentamethylen-diamin, 1,6-Hexamethylen-diamin, 2,2,4-Trimethyl-1,6-hexamethylen-diamin, 1,8-Octamethylen-diamin, 1,10-Decylendiamin, 1,12-Dodecylen-diamin und 3,3'-Diaminodipropylether, cycloaliphatischen Diaminen, wie 1,2-, 1,3- und 1,4-Cyclohexan-diamin, 2,4- und 2,6-Hexahydrotoluylen-diamin sowie die entsprechenden Isomerengemische, 1,4-Hexahydroxylylen-diamin, 4,4'-, 2,4'- und 2,2'-Diamino-dicyclohexylmethan sowie die entsprechenden Isomerengemische, 2,2-Di-(4-aminocyclohexyl)-propan, 3-Aminomethyl-3,5,5-trimethylcyclohexylamin und Dicyclopentadienylverbindungen der Formel

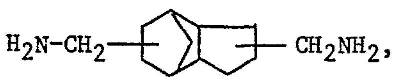

heterocyclische Reste gebunden enthaltenden Diaminen, wie z.B. gegebenenfalls substituierten N,N'-Bis-(aminoalkyl)-piperazinen, z.B. N,N'-Bis-(2,2-dimethyl-3-aminopropyl)-piperazin und N,N'-Bis-(aminopropyl)-piperazin, aromatischen Monoaminen wie Anilin, substituierten Anilinen, wie in 2-, 3- und/oder 4-Stellung durch Nitro-, Methyl-, Ethyl-, n-Propyl-, Isopropyl-, n-Butyl-, Isobutyl-, sek.-Butyl-, tert.-Butylgruppe oder ein Chloratom substituierte Aniline, ortho-, meta- und/oder para-Hydroxy-, Methoxy-, Ethoxy-, Propoxy-, Isopropoxy-, N-Butoxy-, Isobutoxy-, sek.-Butoxy- und tert.-Butoxyanilin; durch eine Aminogruppe in m- und/oder p-Stellung substituierten Benzoesäurealkylestern mit 1 bis 4 Kohlenstoffatomen im Alkylrest, durch eine Aminogruppe in m- und/oder p-Stellung substituierten N-Alkoxycarbonylaminobenzolen und -toluolen mit 1 bis 4 Kohlenstoffatomen im Alkylrest; α- und ß-Naphthylamin; aromatischen Diaminen wie 1,3- und 1,4-Diaminobenzol; durch eine Nitro-, Methyl-, Ethyl-, n-Propyl-, Isopropyl-, n-Butyl-, Isobutyl-, sek.-Butyl-, tert.-Butyl-, Methoxy-, Ethoxy-, n-Propoxy-, Isopropoxy-, n-Butoxy-, Isobutoxy-, sek.-Butoxy-, tert.-Butoxygruppe oder ein Halogenatom, vorzugsweise ein Fluor- und/oder Chloratom, in 2- und/oder 4-Stellung substituiertem 1,4-Diaminobenzol, 1,5- und 1,8-Diaminonaphthalin, 4,4'-Diaminodiphenyl, 2,2'-, 2,4'- und 4,4'-Diaminodiphenylmethan und den entsprechenden Isomerengemischen sowie aromatischen Polyaminen, wie 1,3,5-Triaminobenzol, 2,4,6-Triaminotoluol, 1,3,5-Triaminonaphthalin und Polyphenyl--polymethylen-polyaminen, sowie Mischung aus Diamino-diphenylmethanen und Polyphenyl-polymethylen-polyaminen.

Die nach dem erfindungsgemäßen Verfahren hergestellten Mono- und/oder Polyurethane sind wertvolle End- und Zwischenprodukte. Als Endprodukte werden sie beispielsweise als Schädlingsbekämpfungsmittel verwendet. Als Zwischenprodukte werden sie beispielsweise als Komponenten für Polykondensations- und Polymersysteme eingesetzt, insbesondere jedoch unter Abspaltung von Alkoholen in die entsprechenden Isocyanate übergeführt, wobei Di- und Polyisocyanate zur Herstellung von Polyurethankunststoffen Anwendung finden.

Die in den Beispielen genannten Teile beziehen sich auf das Gewicht. Die Elementarzusammensetzungen und Strukturen sind durch Massenspektroskopie sowie IR und NMR-Spektren bestätigt.

Beispiel 1

Eine Mischung aus 8,6 g N,N'-Diphenylallophansäurebutylester und 15,9 g N-Phenylbutylurethan wurde mit 58,1 g n-Butanol bei 210°C 5 Stunden lang gerührt. Nach dem Abkühlen der Reaktionsmischung destillierte man das überschüssige n-Butanol ab und erhielt nach der Reinigung über einen Dünnfilmverdampfer 25,5 g N-Phenylbutylurethan (90,2 % der Theorie). Der Allophanatumsatz war quantitativ.

Die Mischung der Ausgangskomponenten N,N'-Diphenylallophansäurebutylester und N-Phenylbutylurethan wurde erhalten durch Verrühren mit n-Butanol eines Destillationssumpfes, welcher bei der Reindestillation von Phenylisocyanat anfiel, das seinerseits durch thermische Spaltung von N-Phenylbutylurethan hergestellt wurde.

Beispiel 2

Eine Mischung aus 26 g eines aus 2-Butoxicarbonylamino-toluylen-4-isocyanat und 2,4-Toluylendibutylurethan erhaltenen Allophanats und 14 g 2,4-Toluylendibutylurethan, wie sie durch Verrühren mit n-Butanol eines Destillationssumpfes der Reindestillation von durch thermische Spaltung von 2,4-Toluylendibutylurethan erhaltenen 2,4-Toluylen-diisocyanats anfiel, wurde mit 51,8 g n-Butanol bei 210°C 5 Stunden lang gerührt. Nach dem Abkühlen der Reaktionsmischung destillierte man das überschüssige n-Butanol ab und erhielt einen Rückstand, der nach HPLC-Analyse 42,8 g

2,4-Toluylendibutylurethan (98,0 % der Theorie) enthielt. Allophanat konnte nicht mehr nachgewiesen werden.

Beispiel 3

Bei der Reindestillation eines durch thermische Spaltung von Hexamethylendibutylurethan-1,6 erhaltenen Hexamethylendiisocyanats-1,6 wurden 135 g einer Mischung aus 85 Gew.% 6-(Butoxicarbonylamino)-hexylisocyanat und 15 Gew.% Hexamethylen-diisocyanat-1,6 in der Destillationsblase im Durchschnitt ca. 2 Stunden lang auf Temperaturen von 155 bis 165°C erhitzt. Hierbei erhielt man eine Mischung, die aus ungefähr 18 Gew.% 6-(Butoxicarbonylamino)-hexylisocyanat und verschiedenen Polyisocyanato-hexamethylenallophansäurebutylester bestand.

Zu dieser Mischung fügte man 310 g n-Butanol und 1 g Eisen-(III)-acetat und erhitzte das Gemisch in einer Druckapparatur 5 Stunden lang auf 220°C. Nach dem Abkühlen wurde das Reaktionsgemisch mit Hilfe der Gaschromatographie analysiert. Man erhielt 183 g Hexamethylendibutylurethan-1,6, dies bedeutete, daß die Polyisocyanato-hexamethylenallophansäurebutylester praktisch quantitativ zu Diurethan umgesetzt wurden.

Beispiel 4

Man verfuhr analog den Angaben von Beispiel 3, verwandte jedoch 78 g einer Mischung, die ungefähr 16 Gew.% 6-(Butoxicarbonylamino)-hexylisocyanat erhielt, anstelle von n-Butanol 450 g n-Octanol und 0,5 g Eisen-(III)-acetat.

Die Reaktionsmischung wurde 3 Stunden lang zum Sieden erhitzt, hierbei das abgespaltene n-Butanol abdestilliert und nach dem Abkühlen der Reaktionsmischung das überschüssige n-Octanol unter vermindertem Druck abdestilliert. Man erhielt 153 g eines Rückstandes, der nach der Dünnschichtanalyse praktisch quantitativ aus Hexamethylen-dioctylurethan-1,6 bestand.

Patentansprüche

1. Verfahren zur Herstellung von N,O-substituierten Mono- und/oder Polyurethanen der Formel

$$R^1[NHCOOR^2]_n ,$$

in der

$R^1$ einen gegebenenfalls substituierten aliphatischen, cycloaliphatischen, aromatischen, araliphatischen oder heterocyclischen Rest,

$R^2$ einen gegebenenfalls mit Alkoxy- oder Polyoxyalkylengruppen substituierten aliphatischen, cycloaliphatischen oder araliphatischen Rest, die gleich oder verschieden sind, falls n größer als 1 ist und

n eine ganze Zahl von 1 bis 5 bedeuten, dadurch gekennzeichnet, daß man N-substituierte Allophansäure- und/oder Polyallophansäureester mit Alkoholen in Abwesenheit oder Gegenwart von Katalysatoren bei Temperaturen von mindestens 160°C zur Reaktion bringt.

2. Verfahren nach Anspruch 1, dadurch gekennzeichnet, daß man die Reaktion bei Temperaturen von 165° bis 250°C durchführt.

3. Verfahren nach Anspruch 1, dadurch gekennzeichnet, daß man die Reaktion in Gegenwart von Katalysatoren durchführt.

4. Verfahren nach Anspruch 1, dadurch gekennzeichnet, daß man als Katalysatoren 0,0001 bis 10 Gew.% mindestens eines Kations von Metallen der Gruppen IA, IB, IIA, IIB, IIIA, IIIB, IVA, IVB, VA, VB, VIB, VIIB, VIIIB des Periodensystems, bezogen auf das Gewicht der Allophansäure- und/oder Polyallophansäureester verwendet.

5. Verfahren nach Anspruch 1, dadurch gekennzeichnet, daß man 1 bis 50 Mole Alkohol pro $[CONR^1]$-Gruppe der Allophansäure- und/oder Polyallophansäureester verwendet.

6. Verfahren nach Anspruch 1, dadurch gekennzeichnet, daß man den dem Allophansäure- und/oder Polyallophansäureester entsprechenden Alkohol verwendet.

7. Verfahren nach Anspruch 1, dadurch gekennzeichnet, daß man den Alkohol mit einem unter den Reaktionsbedingungen inerten Lösungsmittel mischt.

8. Verfahren nach Anspruch 1, dadurch gekennzeichnet, daß man als Alkohole lineare oder verzweigte Alkanole mit 1 bis 18 Kohlenstoffatomen, mit Alkoxygruppen mit 1 bis 4 Kohlenstoffatomen oder Polyoxyalkylengruppen substituierte Alkanole mit 2 bis 24 Kohlenstoffatomen, cycloaliphatische Alkohole mit 4 bis 12 Kohlenstoffatomen und/oder araliphatische Alkohole mit 7 bis 15 Kohlenstoffatomen verwendet.

9. Verfahren nach Anspruch 1, dadurch gekennzeichnet, daß man als Alkohole Methanol, Ethanol, n-Propanol, iso-Propanol, n-Butanol, iso-Butanol, n-Pentanol, Hexanol, Ethylhexanol, Heptanol, Octanol, Nonanol, Decanol, Cyclopentanol, Benzylalkohol, Cyclohexanol und/oder 2-Phenylethanol verwendet.

10. Verfahren nach Anspruch 1, dadurch gekennzeichnet, daß die Gruppe $R^1$ bedeutet einen 1- bis 5-wertigen Rest eines
substituierten oder unsubstituierten aliphatischen Kohlenwasserstoffs mit 1 bis 18 Kohlenstoffatomen,
eines substituierten oder unsubstituierten cycloaliphatischen Kohlenwasserstoffs mit 3 bis 18 Kohlenstoffatomen,
eines substituierten oder unsubstituierten aromatischen Kohlenwasserstoffs mit 6 bis 15 Kohlenstoffatomen,
eines substituierten oder unsubstituierten araliphatischen Kohlenwasserstoffs mit 7 bis 34 Kohlenstoffatomen oder
eines substituierten oder unsubstituierten 5- bis 6-gliedrigen Heterocyclen, der zusätzlich noch mit einem Benzolring annelliert sein kann.

Europäisches Patentamt

# EUROPÄISCHER RECHERCHENBERICHT

0133274
Nummer der Anmeldung

EP 84108722.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| D,X | HOUBEN-WEYL "Methoden der organischen Chemie", 4. Auflage, Band VIII, 1952<br>GEORG THIEME VERLAG, Stuttgart, Seiten 203-207<br>* Seite 206 *<br>-- | 1,2,8,9 | C 07 C 125/06 |
| D,A | EP - A2,A3 - 0 059 400 (BAYER AG)<br>* Patentansprüche 1-4; Beispiel 1 *<br>---- | 1-4,7-10 | |
| | | | RECHERCHIERTE SACHGEBIETE (Int. Cl.4) |
| | | | C 07 C 125/00 |

Der vorliegende Recherchenbericht wurde für alle Patentansprüche erstellt.

| Recherchenort | Abschlußdatum der Recherche | Prüfer |
|---|---|---|
| WIEN | 21-09-1984 | HERING |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82